(19) Europäisches Patentamt
European Patent Office
Office européen des brevets

(11) Veröffentlichungsnummer : **0 073 364**
**B1**

(12) # EUROPÄISCHE PATENTSCHRIFT

(45) Veröffentlichungstag der Patentschrift : **21.11.85**

(51) Int. Cl.⁴ : **C 07 D251/70**

(21) Anmeldenummer : **82107161.0**

(22) Anmeldetag : **07.08.82**

(54) **Verfahren zur Herstellung von Perfluoralkylreste enthaltenden Kondensationsprodukten, die so hergestellten Kondensationsprodukte und deren Verwendung.**

(30) Priorität : **22.08.81 DE 3133303**

(43) Veröffentlichungstag der Anmeldung : **09.03.83 Patentblatt 83/10**

(45) Bekanntmachung des Hinweises auf die Patenterteilung : **21.11.85 Patentblatt 85/47**

(84) Benannte Vertragsstaaten : **AT BE CH DE FR GB IT LI NL**

(56) Entgegenhaltungen :
**DE-A- 1 768 939**
**DE-B- 1 208 745**
**US-A- 3 362 782**
**US-A- 3 506 661**
**US-A- 3 510 455**

(73) Patentinhaber : **Chemische Fabrik Pfersee GmbH**
**Färberstrasse 4**
**D-8900 Augsburg (DE)**

(72) Erfinder : **Deiner, Hans**
**Kuglerberg 13**
**D-8902 Neusäss (DE)**
Erfinder : **Mosch, Franz**
**Franz-Beer-Strasse 4 c**
**D-8901 Gessertshausen (DE)**
Erfinder : **Sandner, Bernhard**
**Ortlerweg 12**
**D-8901 Diedorf (DE)**
Erfinder : **Bernheim, Willy, Dr.**
**Beethovenstrasse 28**
**D-8901 Diedorf (DE)**

**EP 0 073 364 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Perfluoralkylreste enthaltenden Kondensationsprodukten, die nach dem Verfahren hergestellten Kondensationsprodukte sowie deren Verwendung in Form von wäßrigen Dispersionen zum Öl- und Wasserabweisendmachen von Fasermaterial, insbesondere von Textilmaterial.

Aus der US-PS 3 510 455 ist es bekannt, wärmehärtbare Kondensationsprodukte aus einem Aldehyd, einer Stickstoffverbindung, wie beispielsweise Harnstoff und Melamin, und einer fluorierten aliphatischen Verbindung, die sowohl eine perfluorierte Alkylkette als auch mindestens eine funktionelle Gruppe mit einem aktiven Wasserstoffatom enthält, herzustellen. Diese Kondensationsprodukte sind in üblichen Lösungsmitteln löslich und härten auf polaren Oberflächen zu einem kontinuierlichen, gut haftenden Film aus. Die Umsetzung erfolgt in anderen Molverhältnissen und bei durchschnittlich relativ niedrigen Temperaturen (siehe Beispiele 1 bis 16). Die Thiole B) werden hier nicht genannt. Zur Behandlung von Textilien sind die nach diesem Stande der Technik erhaltenen Kondensationsprodukte offensichtlich nicht geeignet.

Aus der US-PS 3 362 782 ist es bekannt, Cellulosetextilmaterial mit Kondensationsprodukten aus gegebenenfalls veretherten Methylolverbindungen heterocyclischer Stickstoffverbindungen mit 1,1-Dihydroperfluoralkoholen oder Perfluorcarbonsäureamiden, wie z. B. mit Perfluorheptyloxymethylpenta-(methoxymethyl)-melamin schmutzfest auszurüsten. Die Anwendung erfolgt dabei aus organischem Lösungsmittel. Die mit diesen Kondensationsprodukten erzielbaren Öl- und Wasserabweisungswerte genügen jedoch den heutigen Anforderungen nicht mehr. Darüberhinaus müssen sie in unverhältnismäßig großen Mengen angewandt werden.

Es wurde nun ein Verfahren zur Herstellung von Kondensationsprodukten aus mindestens teilweise veretherten Methylolverbindungen heterocyclischer Stickstoffverbindungen und aliphatischen, einen Perfluoralkylrest und ein aktives Wasserstoffatom enthaltenden Verbindungen gefunden, das dadurch gekennzeichnet ist, daß man

A) 1 Mol eines mit $C_1$- bis $C_4$-Alkoholen hochveretherten Hexamethylolmelamins mit

B) 3 bis 6 Mol eines Thiols der allgemeinen Formel

$$R_f\text{—}X\text{—}SH \qquad (I),$$

worin $R_f$ einen perfluorierten Alkylrest mit 4 bis 14 C-Atomen und X den Ethylen- oder Isopropylenrest bedeuten, unter Temperatursteigerung auf mindestens 145 °C während mindestens 40 Minuten miteinander umsetzt.

Gegenstand der vorliegenden Erfindung sind ferner die nach diesem Verfahren hergestellten Kondensationsprodukte sowie deren Verwendung in Form von wäßrigen Dispersionen zum Öl- und Wasserabweisendmachen von Fasermaterial, insbesondere von Textilmaterial. Die nach dem erfindungsgemäßen Verfahren hergestellten Kondensationsprodukte ergeben nämlich auf Fasermaterialien öl- und wasserabweisende Eigenschaften, die denjenigen des erwähnten Standes der Technik überlegen sind.

Aufgrund des Standes der Technik konnte nicht erwartet werden, daß Kondensationsprodukte, bei denen ausgewählte Verbindungen, nämlich hochverethertes Hexamethylolmelamin A) und das Thiol B) im Molverhältnis 1 : 3 bis 6, insbesondere bei 1 : 4 bis 5,5 miteinander umgesetzt worden sind, ganz besonders günstige Eigenschaften hinsichtlich der Öl- und Wasserabweisung auf Fasermaterial ergeben würden.

Die als Ausgangsprodukte verwendeten hochveretherten Hexamethylolmelamine sind bekannt. Sie sind beispielsweise nach dem Verfahren der GB-PS 611 013 zugänglich. Unter den hochveretherten Hexamethylolmelaminen sind solche zu verstehen, in denen das Melamin vollständig methyloliert ist und die Methylolgruppen weitgehend verethert sind. Sehr gut als Ausgangsprodukt brauchbar ist z. B. ein Hexamethylolmelaminether, dessen Methylolgruppen zu 88 bis 95 Mol% mit einem Alkohol mit 1 bis 4, insbesondere mit 1 bis 3 C-Atomen, verethert sind. Aus ökonomischen Gründen wird der entsprechende Methylether besonders bevorzugt, im folgenden mit Hexamethylolmelaminhexamethylether bezeichnet. Die vollständige Methylolierung und der hohe Veretherungsgrad sind deshalb wichtig, da andernfalls das Melaminderivat A) für die Umsetzung mit dem Thiol B) nicht hinreichend beständig ist.

Auch die als 2. Ausgangskomponente B) eingesetzten aliphatischen Thiole mit Perfluoralkylrest der allgemeinen Formel

$$R_f\text{—}X\text{—}SH \qquad (I),$$

worin $R_f$ einen perfluorierten Alkylrest mit 4 bis 14 C-Atomen und X den Ethylen- bzw. Isopropylenrest bedeuten, sind bekannt (US-PS 3 088 849 und DE-OS 20 13 103 entsprechend ZA-Anmeldung 71 01 739). Die Herstellung gelingt durch Umsetzung von Perfluoralkylhalogeniden mit Thioharnstoff und anschließende Verseifung mit Alkali oder wäßrigen Lösungen von Ammoniak. Von den Verbindungen B) werden aus praktischen Gründen diejenigen Thiole bevorzugt, in denen X den Ethylenrest bedeutet.

Da die aus den beiden Komponenten, nämlich dem hochveretherten Hexamethylolmelamin A) und

dem Thiol B) hergestellten Kondensationsprodukte zum Öl- und Wasserabweisendmachen von Fasermaterial, insbesondere von Textilmaterial, verwendet werden, sind zur Herstellung die Thiole bevorzugt, bei welchen der perfluorierte Alkylrest mindestens 6 C-Atome aufweist. Die Verbindung B) muß nicht in reiner Form vorliegen. Bevorzugt werden sogar technische Gemische eingesetzt, in welchen der perfluorierte Alkylrest überwiegend 6, 8 und 10 C-Atome, also durchschnittlich 7 bis 9 C-Atome aufweist.

In Betracht kommen Umsetzungsprodukte, bei denen das Molverhältnis von A) zu B) 1 : 3 bis 6, insbesondere 1 : 4 bis 5,5 beträgt. Es ist dabei durchaus möglich, mit einem Überschuß an Fluorverbindung B) zu arbeiten. Dieser wird aber am Schluß der Umsetzung, gegebenenfalls im Vakuum, wieder abgezogen.

Die Kondensation wird in der Weise vorgenommen, daß man die beiden Komponenten in den gewünschten Molverhältnissen mischt und in einem mit Rührer, Gaseinleitungsrohr, Innenthermometer und absteigenden Kühler ausgestattetem 4-Halskolben unter Rühren, zweckmäßig in einer Schutzgasatmosphäre unter Temperatursteigerung auf mindestens 145 °C miteinander umsetzt. Dazu wird die Temperatur vorzugsweise stufenweise erhöht, und zwar wird innerhalb von ca. 15 bis 45 Minuten bevorzugt möglichst gleichmäßig auf eine Temperatur von 95 bis 130 °C erhitzt, dann die Temperatur innerhalb von weiteren 25 bis 120 Minuten, insbesondere 60 bis 120 Minuten, auf 145 bis 200 °C, insbesondere auf 160 bis 180 °C gesteigert und dann vorteilhaft abschließend noch weitere 2 bis 6 Stunden bei dieser Temperatur belassen. Während der Kondensation destilliert der durch die « Umetherungsreaktion » frei werdende leicht flüchtige Alkohol ab. Die Ausbeute, bestimmt durch Zurückwägen des zuvor tarierten Kolbens, beträgt ca. 92 bis 98 % der Theorie.

Wird die letzte Phase der Erhitzungsdauer bei niedrigerer Temperatur als 145 °C durchgeführt, so zeigen die erhaltenen Kondensationsprodukte in ihrer Anwendung zwar noch befriedigende Ölabweisungswerte, doch lassen die Werte für die Wasserabweisung zu wünschen übrig.

Die Kondensation kann in Gegenwart eines Umetherungskatalysators, insbesondere eines sauren Katalysators durchgeführt werden. Als solche kommen vor allem schwerflüchtige anorganische oder organische Säuren, wie Phosphorsäure, p-Toluolsulfosäure oder Oxalsäure in Mengen von 0,05 bis 0,5, insbesondere von 0,1 bis 0,25 Gew.% bezogen auf die gesamte Reaktionsmasse, in Betracht. Im Prinzip ist es möglich, die Kondensation auch in einem Lösungsmittel, das einen genügend hohen Siedepunkt aufweist, durchzuführen.

Nach Erhitzung und Abkühlung auf etwa 70 bis 90 °C wird das Kondensationsprodukt in etwa der dreifachen Gewichtsmenge eines geeigneten Lösungsmittels bevorzugt warm gelöst. Die erhaltenen Umsetzungsprodukte sind in den herkömmlichen organischen Lösungsmitteln bei Normaltemperatur nicht mehr löslich, lassen sich aber in Form ihrer Lösungen in Fluorkohlenwasserstoffen, wie 1,1,2-Trichlor-1,2,2-trifluorethan, Benzotrifluorid oder 1,3-Bis-(trifluormethyl)-benzol in üblicher Weise unter Zusatz von Emulgatoren gut in beständige, wäßrige Emulsionen überführen.

Die Emulgatoren sind bekannt. Als solche kommen beispielsweise in Betracht:

Polyvinylalkohol in wäßriger Lösung, ethoxylierte Fettsäureamide, ethoxylierte Fettsäuren, ethoxylierte Fettalkohole und ethoxylierte Fettamine, letztere auch in Form ihrer Salze mit niedermolekularen organischen Säuren oder Mineralsäuren, sowie quaternäre Ammoniumverbindungen, wie Octadecyloxymethylpyridiniumchlorid oder Cetylbenzyldimethylammoniumchlorid. Ebenfalls als Emulgator geeignet sind Umsetzungsprodukte aus Epoxygruppen enthaltenden Verbindungen und Di- oder Polyaminen, wie sie z. B. in der US-PS 3 320 197 beschrieben sind, oder Kondensationsprodukte aus Harnstoff und seinen Derivaten mit Epoxygruppen enthaltenden Verbindungen und ausgewählten Aminen, wie sie in der US-PS 3 729 437 beschrieben sind.

Die Emulgatoren werden dabei in üblichen Mengen von 4 bis 30, insbesondere 8 bis 25 Gew.% (gerechnet als Festsubstanz), bezogen auf das lösungsmittelfreie Kondensationsprodukt, eingesetzt.

Es ist zweckmäßig, nach der Emulgierung das Lösungsmittel durch an sich bekannte Maßnahmen zu entfernen, da hierdurch bei der Anwendung des Produktes eine Verunreinigung der Luft mit den Lösungsmitteldämpfen vermieden wird und zudem das zurückgewonnene Lösungsmittel wieder verwendet werden kann.

Die wäßrigen Dispersionen werden in der Regel kombiniert mit in der Textilindustrie üblichen Hilfsmitteln angewandt. Hervorzuheben sind hier Mittel, mit welchen die Knitterfestigkeit verbessert wird. Zur weiteren Verbesserung, vor allem der Hydrophobiereffekte, werden die Kondensationsprodukte bevorzugt mit sogenannten Extendern angewandt. Solche Extender, also Stoffe, die die Öl- und Wasserabweisung verbessern und dabei eine Verringerung der Menge der Fluoralkylreste enthaltenden Verbindungen ermöglichen, sind z. B. die entsprechend der US-PS 3 506 661 oder in ähnlicher Weise hergestellten fettmodifizierten Kunstharze in Emulsionsform und die bekannten Metallsalz/Paraffinemulsionen.

Ferner kommen als Hilfsmittel noch solche in Betracht, die die Flammfestigkeit oder die Verrottungsfestigkeit verbessern, Appreturmittel, die den zu behandelnden Textilien einen gewünschten Griff verleihen, und andere. Diese Mittel sind alle dem Fachmann geläufig. Allgemein gilt, daß die üblichen Hilfsmittel, soweit erforderlich, auch mit den bekannten Härtungskatalysatoren eingesetzt werden.

Die Behandlung mit den erfindungsgemäßen Kondensationsprodukten ist für Fasermaterialien aller Art geeignet. Als Fasermaterialien sind insbesondere Textilien in Form von Geweben, Gewirken oder gegebenenfalls vorverfestigten Faservliesen und ferner Leder, Kunstleder und Papier aus den üblichen

Fasermaterialien zu nennen. Das bevorzugt zu behandelnde Textilmaterial ist bekannt. Es besteht aus natürlichen Fasern, wie Cellulose oder Wolle bzw. aus synthetischen Fasern, wie Polyester, Polyamid oder Polyacrylnitril. Darüberhinaus können selbstverständlich auch Textilmaterialien behandelt werden, die aus Mischungen von natürlichen mit synthetischen Fasern hergestellt werden.

Die Behandlung wird in bekannter Weise vorgenommen. Beispielsweise werden die Textilien mit den wäßrigen Behandlungsflotten foulardiert, auf eine Flottenaufnahme von ca. 60 bis 100 Gew.% abgequetscht, bei 80 bis 110 °C vorgetrocknet und einige Minuten, insbesondere 1 bis 8 Minuten, bei etwa 130 bis 170 °C ausgehärtet. Selbstverständlich sind auch andere Auftragsmethoden, wie Sprühen oder Pflatschen, geeignet, wobei die Einsatzmengen entsprechend der Flottenaufnahme zu variieren sind.

Die Vorteile der Erfindung liegen vor allem darin, daß mit relativ geringen Mengen an Perfluoralkylgruppen enthaltenden Kondensationsprodukten auf dem Fasermaterial überraschend hohe Ölabweisungswerte, verbunden mit einem hohen Grad an Wasserabweisung erzielt werden können, wobei diese Eigenschaften auch nach mehrfachen Wäschen und Lösungsmittelbehandlungen (Trockenreinigung) weitgehend erhalten bleiben. Es war keineswegs vorherzusehen, daß durch die aufgeführten Reaktionsbedingungen eine so erhebliche Effektsteigerung erreicht werden würde, wobei auch von entscheidender Bedeutung für die erfindungsgemäß hergestellten Kondensationsprodukte die Wahl des richtigen Umsetzungsverhältnisses von hochverethertem Hexamethylolmelamin A) zu dem Thiol B) ist.

Diese sehr guten Eigenschaften konnten aufgrund des Standes der Technik nicht erwartet werden.

In den folgenden Beispielen erfolgt die Prüfung der Ölabweisung nach der Methode, wie sie in AATCC 118 — 1972 angegeben ist. Die Prüfung der Wasserabweisung erfolgt nach DIN 53 888 (a = Wasseraufnahme in % ; b = Abperleffekt) bzw. dem Spray-Test entsprechend AATCC 22 — 1974. Die Trockenreinigung erfolgt im Flottenverhältnis 1 : 10 (Mustergewicht zu Flottenvolumen) während 15 Minuten, wobei für jede Reinigung das verwendete Tetrachlorethylen erneuert wird. Gewaschen werden die Muster unter Verwendung eines Vollwaschmittels im üblichen Maschinenwaschgang bei den in den Beispielen angegebenen Temperaturen.

## Beispiel 1

### Herstellung des Kondensationsproduktes

In einem 1 Liter-4-Halskolben — ausgestattet mit Rührer, Gaseinleitungsrohr, Innenthermometer und Destillieraufsatz mit absteigendem Kühler und Vorlage — werden 233,3 g (ca. 0,5 Mol) $R_fCH_2CH_2SH$ ($R_f$ bedeutet zu 1,3 Gew.% $C_4F_9$-, 34,3 Gew.% $C_6F_{13}$-, 31,8 Gew.% $C_8F_{17}$-, 22,5 Gew.% $C_{10}F_{21}$-, 8,2 Gew.% $C_{12}F_{25}$- und zu 1,9 Gew.% $C_{14}F_{29}$-),

39,0 g (ca. 0,1 Mol) Hexamethylomelaminhexamethylether und

0,5 g p-Toluolsulfosäurehydrat

gemischt und unter Rühren und Einleiten eines schwachen Stickstoffstroms gleichmäßig innerhalb von 30 Minuten auf 115 °C und während weiterer 90 Minuten bis auf 175 °C erhitzt. Danach werden diese Bedingungen während 6 Stunden beibehalten. Dabei destilliert Methanol mit Spuren an Perfluorverbindung ab.

Anschließend wird auf etwa 80 °C abgekühlt und die Ausbeute durch Wiegen bestimmt (95 % der Theorie). Das noch warme Kondensationsprodukt wird in 730 g Benzotrifluorid gelöst.

### Emulgierung des Kondensationsproduktes

500 g der erhaltenen 25 %igen Kondensationsproduktlösung und 1 000 g Wasser, das 2,7 Gew.% Emulgator (Hydroxialkylaminpolyglykoletheracetat mit insgesamt 10 Mol Ethylenoxyd) enthält, werden mit einem Schnellrührer voremulgiert und durch Hochdruckhomogenisierung (bei 250 bar und maximal 50 °C) emulgiert. Aus dieser Emulsion wird das Lösungsmittel im Wasserstrahlvakuum zusammen mit Wasser bei bis zu 50 °C ausdestilliert und eine an Kondensationsprodukt 12,4 %ige Emulsion erhalten.

### Öl- und wasserabweisende Ausrüstung

Mit einer Flotte enthaltend

30 g/l der obigen Emulsion

10 g/l Aminoplastharzlösung (ca. 52 %ige wäßrige Lösung von Dimethylolethylenharnstoff und mit $CH_3OH$ verethertem Pentamethylolmelamin im Verhältnis 2,5 : 1)

30 g/l Extender (etwa 16 %ige handelsübliche Metallsalz/Paraffinemulsion)

1 ml/l Katalysatorlösung (ca. 75 %ige wäßrige Zinknitrathexahydratlösung mit geringen Mengen Salz- und Essigsäure)

30 ml/l eines handelsüblichen Netzmittels auf Basis Isoprapanol und Isobutanol und

2 ml/l 60 %ige Essigsäure, werden A) ein Polyacrylnitrilgewebe (214 g/m²) und B) ein Wolle/Polyestermischgewebe (65/35 ; 308 g/m²)

foulardiert (Flottenaufnahme 90 bzw. 70 %), 10 Minuten bei 110 °C getrocknet und 5 Minuten bei 150 °C kondensiert.

Die erhaltenen Effekte und deren Permanenz sind der nachgolgenden Tabelle zu entnehmen (a = Wasseraufnahme in % ; b = Abperleffekt) :

Hydrophobiereffekte

| Gewebe | Wäsche | Original | | nach 3 Wäschen bei $40^{\circ}C$ | | nach 3 Reinigungen | |
|---|---|---|---|---|---|---|---|
| | | a | b | a | b | a | b |
| A | 3 x $40^{\circ}C$ | 11 | 5/5/5 | 8 | 4/4/4 | 8 | 4/4/4 |
| B | 3 x $40^{\circ}C$ | 11 | 5/4/4 | 19 | 4/4/3 | 12 | 4/4/4 |

Ölabweisung

| | Original | nach 3 Wäschen bei $40^{\circ}C$ | nach 3 Reinigungen |
|---|---|---|---|
| A | 6 | 6 | 6 |
| B | 6 | 6 | 6 |

Für die unbehandelten Gewebe wurden folgende Werte gemessen :
A : a = 107 ; b = 1 ; Ölabweisung O ; B : a = 42 , b = 1 ; Ölabweisung O.

Beispiel 2

Emulsion A

Entspricht der im Beispiel 1 hergestellten Emulsion.

Emulsion B

Entsprechend den Angaben im Beispiel 1 wird ein Kondensationsprodukt unter Verwendung eines mit Ethanol hochveretherten Hexamethylolmelamins hergestellt und wie dort angegeben in eine beständige Emulsion (12,5 Gew.% Kondensationsprodukt) überführt.

Emulsion C

Entsprechend den Angaben im Beispiel 2 der US-PS 3 510 455 wird ein Kondensationsprodukt unter Verwendung von 1 Mol $C_8F_{17}SO_2N(C_2H_5)CH_2CH_2OH$ und 1 Mol Hexamethylolmelaminhexamethylether hergestellt und wie im Beispiel 1 beschrieben zu einer wäßrigen Emulsion (ca. 12 Gew.% Kondensationsprodukt) verarbeitet.

Emulsion D

Entsprechend den Angaben in Spalte 7, Zeilen 38 ff der US-PS 3 362 782 wird ein Kondensationsprodukt unter Verwendung von 1 Mol Perfluoralkyltetrahydroalkohol (Perfluoralkylrest mit durchschnittlich 7 bis 8 C-Atomen) und 1 Mol Hexamethylolmelaminhexamethylether hergestellt und wie im Beispiel 1 beschrieben zu einer wäßrigen Emulsion (ca. 12 Gew.% Kondensationsprodukt) verarbeitet.

Emulsion E

Vergleichsweise werden die in Beispiel 1 genannten Verbindungen im Molverhältnis 1 : 1 umgesetzt und in eine wäßrige Emulsion (ca. 12 Gew.% Kondensationsprodukt) überführt.

Mit diesen Emulsionen wird ein blauer Baumwollköper (222 g/m²) entsprechend den Angaben im Beispiel 1 ausgerüstet, wobei die Emulsionen A bis E jeweils in Mengen entsprechend 2,25 g Fluor/Liter zum Einsatz kommen (30 g/l Emulsion A, 30 g/l Emulsion B, 55 g/l Emulsion C, 48 g/l Emulsion D bzw. 50 g/l Emulsion E) und im übrigen folgende Bestandteile enthalten :

50 g/l der im Beispiel 1 genannten Aminoplastharzlösung,

5 ml/l der im Beispiel 1 genannten Katalysatorlösung,
2 ml/l 60 %ige Essigsäure und
20 g/l eines handelsüblichen Extenders (ca. 20 %ige Emulsion eines entsprechend Beispiel 1 der US-PS 3 506 661 hergestellten modifizierten Melaminethers).

Die technologische Prüfung ergibt für die Öl- und Wasserabweisung folgende Werte :

| Emulsion | | Original | | | nach 3 Reinigungen | |
|---|---|---|---|---|---|---|
| | | a | b | Ölabweisung | Spray-Test | Ölabweisung |
| A) | erfindungsgemäß | 5 | 5/5/5 | 5 | 3 x 100 | 4 |
| B) | | 6 | 5/5/5 | 5 | 3 x 100 | 4 |
| C) | Stand der Technik | 32 | 1 | 4 | 3 x 80 | 0 |
| D) | | 32 | 1 | 4 | 80/70/70 | 0 |
| E) | Vergleich | 29 | 1 | 4 | 80/80/70 | 0 |
| | unbehandelt | 103 | 1 | 0 | - | - |

Die Überlegenheit des erfindungsgemäßen Verfahrens ist überaus deutlich.

## Beispiel 3

### Herstellung des Kondensationsproduktes

In der im Beispiel 1 beschriebenen Apparatur werden
0,4 Mol (188 g) $R_fCH_2CH_2SH$ ($R_f$ bedeutet zu 8,05 Gew.% $C_6F_{13}$- und zu 91,9 Gew.% $C_8F_{17}$-),
0,1 Mol (ca. 54 g) mit n-Propanol weitgehend verethertes Hexamethylolmelamin und
0,5 g Oxalsäure
gemischt und unter Rühren und Einleiten eines schwachen Stickstoffstroms innerhalb von 40 Minuten auf 125 °C und während weiterer 105 Minuten bis auf 185 °C erhitzt. Unter diesen Bedingungen wird der Ansatz 5 Stunden gehalten, wobei n-Propanol und Spuren der Perfluorverbindung abdestillieren.

Anschließend wird auf 75 °C abgekühlt und das zu 93 % der Theorie erhaltene Kondensationsprodukt warm in der 3-fachen Gewichtsmenge (608 g) Benzotrifluorid gelöst.

### Emulgierung des Kondensationsproduktes

In der im Beispiel 1 beschriebenen Weise wird eine an Kondensationsprodukt 12 Gew.%ige Emulsion hergestellt, wobei als Emulgator 150 g einer 10 %igen Polyvinylalkohollösung (Polyvinylalkohol mit einer Verseifungszahl von 140 und einer Viskosität von 25 mPa.s bei 20 °C in 4 %iger Lösung) bei entsprechend reduziertem Wasserzusatz verwendet und das Lösungsmittel bei vermindertem Druck bei bis zu 45 °C entfernt wird.

### Öl- und wasserabweisende Ausrüstung

Mit einer Flotte enthaltend neben den im Beispiel 2 genannten Produkten 34 g/l der obigen Emulsion (anstelle der dort genannten Emulsionen A bis E) wird unter den im Beispiel 1 genannten Bedingungen ein Baumwollköper (222 g/m²) ausgerüstet (Flottenaufnahme 65 %) und dabei werden folgende Effekte erhalten :

Hydrophobiereffekte

| Original | | nach 3 Maschinenwäschen bei 60 °C | nach 3 Reinigungen |
|---|---|---|---|
| a | b | Spray-Test | Spray-Test |
| 5 | 5/5/5 | 3 x 100 | 3 x 100 |

Ölabweisung

| Original | nach 3 Maschinenwäschen bei 60 °C | nach 3 Reinigungen |
|---|---|---|
| 4 | 4 | 4 |

In gleicher Weise kann ein Kondensationsprodukt unter Verwendung eines mit n-Butanol weitgehend veretherten Hexamethylolmelamins hergestellt werden. Wird die entsprechend hergestellte Emulsion zur Ausrüstung des gleichen Gewebes herangezogen, so ist bei etwa gleichen Originalwerten die Wasch- und Reinigungsbeständigkeit etwas schwächer ausgeprägt.

## Beispiel 4

### Herstellung der Kondensationsprodukte

In der im Beispiel 1 beschriebenen Apparatur werden
A) 0,35 Mol (168 g) bzw.
B) 0,45 Mol (216 g) bzw.
C) 0,6 Mol (288 g)
$R_fCH_2CH_2SH$ (technisches Gemisch, wobei $R_f$ durchschnittlich den Rest $C_8F_{17}$- darstellt) mit jeweils etwa 0,1 Mol (39 g) Hexamethylolmelaminhexamethylether in der im Beispiel 1 beschriebenen Weise umgesetzt, wobei nur 2 Stunden nacherhitzt wird.

Nach dem Abkühlen auf 75 bis 85 °C werden durch Zurückwiegen die Ausbeuten bestimmt (95 bis 96 % der Theorie) und durch Aufnehmen in 560 g bzw. 685 g bzw. 885 g Benzotrifluorid jeweils eine ca. 25 %ige Lösung hergestellt.

### Emulgierung der Kondensationsprodukte

Aus den Lösungen der Kondensationsprodukte A), B) und C) werden in bekannter Weise die Emulsionen A), B) und C) bereitet (siehe Beispiel 1), wobei das Wasser als Emulgator 2 Gew.% ethoxyliertes Stearinsäureamid (12 Mol Ethylenoxyd je Mol Stearinsäureamid) enthält (Gehalt an Kondensationsprodukt ca. 12,5 %).

### Öl- und wasserabweisende Ausrüstung

Der im Beispiel 3 genannte Baumwollköper wird mit folgenden Flotten foulardiert :
A) 32 g/l Emulsion A, übrige Bestandteile wie in Beispiel 2,
B) 30 g/l Emulsion B, übrige Bestandteile wie im Beispiel 2,
C) 28 g/l Emulsion C, übrige Bestandteile wie im Beispiel 2, (Flottenaufnahme ca. 65 %), 10 Minuten bei 100 °C getrocknet und 3 Minuten bei 160 °C kondensiert.

Die erhaltenen Effekte sind in der folgenden Tabelle zusammengestellt :

| Emulsion | | A | B | C |
|---|---|---|---|---|
| Molverhältnis | | 1 : 3,5 | 1 : 4,5 | 1 : 6 |
| a | | 10 | 7 | 8 |
| b | | 5/5/4 | 5/5/5 | 5/5/5 |
| Ölabweisung | | 5 | 5 | 5 |
| Spray-Test | ) nach ) 3 Rei- ) ni- | 3 x 100 | 3 x 100 | 3 x 80 |
| Ölabweisung | ) gungen | 5 | 5 | 3 |

Mit der Emulsion B) kann in gleicher Weise ein Baumwoll/Polyester-Mantelpopeline (35/65 ; 208 g/m²) ausgerüstet werden, wobei nach Ausliegen im Normalklima folgende Effekte resultieren :

| a | b | Ölabweisung | a | b | Ölabweisung | a | b | Ölabweisung |
|---|---|---|---|---|---|---|---|---|
| (Original) | | | (nach 3 Wäschen bei 60°C) | | | (nach 3 Reinigungen) | | |
| 4 | 5/5/5 | 6 | 10 | 4/4/4 | 6 | 16 | 4/3/2 | 6 |

Beispiel 5

Herstellung des Kondensationsproduktes

In der im Beispiel 1 beschriebenen Apparatur werden unter den dort beschriebenen Bedingungen aber ohne Nacherhitzung

233 g (ca. 0,5 Mol) $R_fCH_2CH_2SH$ (Bedeutung von $R_f$ siehe Beispiel 1) und

39 g (ca. 0,1 Mol) Hexamethylolmelaminhexamethylether miteinander umgesetzt.

In gleicher Weise kann die entsprechende Perfluorverbindung der Formel

$$R_fCH_2\underset{\displaystyle CH_3}{\underset{|}{CH}}SH$$

($R_f$ siehe Beispiel 4)
zur Reaktion gebracht werden.

Auch ist es möglich, ohne $N_2$-Spülung zu arbeiten.

Nach dem Abkühlen auf ca. 75 °C wird die Ausbeute durch Zurückwiegen zu 248,3 g bestimmt (97 % der Theorie). Das noch warme Kondensationsprodukt wird in 745 g 1,1,2-Trichlor-1,2,2-trifluorethan gelöst.

Emulgierung des Kondensationsproduktes

Die Emulgierung wird wie im Beispiel 3 beschrieben vorgenommen (ca. 12 % Kondensationsprodukt).

Öl- und wasserabweisende Ausrüstung

Mit einer Flotte enthaltend

30 g/l der obigen Emulsion,

50 g/l Aminoplastharzlösung (ca. 60 %ige wäßrige Lösung von mit $CH_3OH$ verethertem Pentamethylolmelamin),

10 ml/l Katalysatorlösung (40 %ige wäßrige 2-Amino-2-methylpropanolhydrochloridlösung),

2 ml/l Essigsäure 60 % und

20 g/l eines handelsüblichen Extenders (siehe Beispiel 2)

wird A) ein Polyamidtaft (66 g/m²) und

B) der blaue Baumwollköper (siehe Beispiel 2) foulardiert (Flottenaufnahme ca. 65 %), kurz bei 105 °C getrocknet und dann 5 Minuten bei 150 °C kondensiert.

Die ausgerüsteten Gewebe zeigen sehr gute Wasser- und Ölabweisung (A : a = 7 ; b = 4/4/4, Ölabweisung = 6 ; B : a = 8 ; b = 5/5/4, Ölabweisung = ), die auch gegen Wäschen und Reinigungen gut beständig sind.

In allen Beispielen erfolgt die Ausrüstung aus wäßrigen Flotten.

Die Prozentangaben beziehen sich auf Gewichtsprozent.

**Patentansprüche**

1. Verfahren zur Herstellung von Kondensationsprodukten aus mindestens teilweise veretherten Methylolverbindungen heterocyclischer Stickstoffverbindungen und aliphatischen, einen Perfluoralkylrest und ein aktives Wasserstoffatom enthaltenden Verbindungen, dadurch gekennzeichnet, daß man

A) 1 Mol eines mit $C_1$- bis $C_4$-Alkoholen hochveretherten Hexamethylolmelamins mit

B) 3 bis 6 Mol eines Thiols der allgemeinen Formel

$$R_f\text{—}X\text{—}SH \qquad (I),$$

worin $R_f$ einen perfluorierten Alkylrest mit 4 bis 14 C-Atomen und X den Ethylen- oder Isopropylenrest bedeuten, unter Temperatursteigerung auf mindestens 145 °C während mindestens 40 Minuten miteinander umsetzt.

2. Verfahren nach Patentanspruch 1, dadurch gekennzeichnet, daß man die Umsetzung unter stufenweiser Temperatursteigerung, und zwar innerhalb von 15 bis 45 Minuten auf eine Temperatur von 95 bis 130 °C und innerhalb von weiteren 25 bis 120 Minuten auf eine Temperatur von 145 bis 200 °C, insbesondere 160 bis 180 °C, vornimmt.

3. Verfahren nach Patentanspruch 2, dadurch gekennzeichnet, daß die Temperatur von 145 bis 200 °C, insbesondere 160 bis 180 °C, noch weitere 2 bis 6 Stunden belassen wird.

4. Verfahren nach Patentanspruch 1 bis 3, dadurch gekennzeichnet, daß man die Verbindungen A) und B) in einem Molverhältnis 1 : 4 bis 5,5 umsetzt.

5. Die nach dem Verfahren der Patentansprüche 1 bis 4 hergestellten Kondensationsprodukte.

6. Verwendung der nach den Patentansprüchen 1 bis 4 hergestellten Kondensationsprodukte in Form von wäßrigen Emulsionen zum Öl- und Wasserabweisendmachen von Fasermaterial.

7. Verwendung nach Patentanspruch 6, dadurch gekennzeichnet, daß als Fasermaterial Textilmaterial eingesetzt wird.

**Claims**

1. Process for the preparation of condensation products from at least partially etherified methylol compounds of heterocyclic nitrogen compounds and aliphatic compounds containing a perfluoroalkyl radical and an active hydrogen atom, characterized in that

A) 1 mol of a highly etherified hexamethylol melamine with $C_1$- to $C_4$-alcohols is reacted with

B) 3 to 6 mol of a thiol of the general formula

$$R_f—X—SH \qquad (I),$$

in which $R_f$ denotes a perfluorinated alkyl radical with 4 to 14 C-atoms and X denotes the ethylene- or isopropylene radical, with a temperature increase to at least 145 °C for at least 40 minutes.

2. Process according to Claim 1, characterized in that the reaction is carried out with a temperature rise in stages, and namely within from 15 to 45 minutes to a temperature of 95 to 130 °C and within a further 25 to 120 minutes to a temperature of 145 to 200 °C, in particular 160 to 180 °C.

3. Process according to Patent Claim 2, characterized in that the temperature of 145 to 200 °C, in particular 160 to 180 °C, is maintained for a further 2 to 6 hours.

4. Process according to Patent Claim 1 to 3, characterized in that the compounds A) and B) are reacted in a molar ratio of 1 : 4 to 5.5.

5. The condensation products prepared according to the process of Patent Claims 1 to 4.

6. Applications of the condensation products prepared according to Patent Claims 1 to 4, in the form of aqueous emulsions for making fibrous material oil- and water-repellent.

7. Application according to Patent Claim 6, characterized in that textile material is used as fibrous material.

**Revendications**

1. Procédé pour la préparation de produits de condensation à partir de composés méthylolés au moins partiellement éthérifiés de composés azotés hétérocycles et de composés aliphatiques contenant un reste perfluoroalkyle et un atome d'hydrogène actif, caractérisé en ce qu'on fait réagir pendant au moins 40 minutes avec élévation de la température jusqu'à au moins 145 °C,

A) 1 mole d'une hexaméthylolmélamine fortement éthérifiée avec des alcools en $C_1$ à $C_4$ avec

B) 3 à 6 moles d'un thiol de formule générale

$$R_f—X—SH \qquad (I)$$

dans laquelle $R_f$ représente un reste alkyle perfluoré avec 4 à 14 atomes de carbone et X le reste éthylène ou isopropylène.

2. Procédé selon la revendication 1, caractérisé en ce qu'on effectue la réaction avec élévation de la température par paliers, à savoir jusqu'à une température de 95 à 130 °C en l'espace de 15 à 45 minutes et jusqu'à une température de 145 à 200 °C, en particulier de 160 à 180 °C en l'espace de 25 à 120 minutes supplémentaires.

3. Procédé selon la revendication 2, caractérisé en ce que la température de 145 à 200 °C, en particulier de 160 à 180 °C est maintenue pendant encore 2 à 6 heures supplémentaires.

4. Procédé selon les revendications 1 à 3, caractérisé en ce qu'on fait réagir les composés A) et B) dans un rapport molaire de 1 : 4 à 5,5.

5. Les produits de condensation préparés selon le procédé des revendications 1 à 4.

6. Utilisation des produits de condensation préparés selon les revendications 1 à 4 sous forme d'émulsions aqueuses pour l'oléofugation et l'hydrofugation de matières fibreuses.

7. Utilisation selon la revendication 6, caractérisé en ce que des matières textiles sont mises en œuvre en tant que matières fibreuses.